Europäisches Patentamt

European Patent Office

Office européen des brevets

(19)

(11) Veröffentlichungsnummer : **0 091 428**
**B1**

(12) **EUROPÄISCHE PATENTSCHRIFT**

(45) Veröffentlichungstag der Patentschrift :
30.12.86

(51) Int. Cl.⁴ : **A 61 M 16/00**

(21) Anmeldenummer : **81902840.8**

(22) Anmeldetag : **21.10.81**

(86) Internationale Anmeldenummer :
**PCT/EP 81/00167**

(87) Internationale Veröffentlichungsnummer :
**WO/8301386 (28.04.83 Gazette 83/10)**

(54) **PUMPELEMENT EINER VORRICHTUNG ZUR KÜNSTLICHEN BEATMUNG.**

(43) Veröffentlichungstag der Anmeldung :
**19.10.83 Patentblatt 83/42**

(45) Bekanntmachung des Hinweises auf die Patenterteilung : **30.12.86 Patentblatt 86/52**

(84) Benannte Vertragsstaaten :
**DE FR GB NL SE**

(56) Entgegenhaltungen :
**FR-A- 1 161 194**
**FR-A- 2 012 494**
**FR-A- 2 266 088**
**GB-A- 1 550 720**
**US-A- 3 473 529**

(73) Patentinhaber : **GOTTLIEB WEINMANN GERÄTE FÜR MEDIZIN UND ARBEITSSCHUTZ GMBH & CO.**
**Kronsaalsweg 40**
**D-2000 Hamburg 54 (DE)**

(72) Erfinder : **BLUMENSAADT, Hans Christian**
**Furesokrogen 8**
**DK-2830 Virum (DK)**

(74) Vertreter : **Liebelt, Rolf, Dipl.-Ing.**
**Glockengiesserwall 2-4**
**D-2000 Hamburg 1 (DE)**

EP 0 091 428 B1

## Beschreibung

Die Erfindung betrifft ein handbetätigtes Pumpelement einer Vorrichtung zur künstlichen Beatmung von Menschen, das aus einem gasdichten, elastischen, selbstexpandierenden und vorzugsweise langgestreckten Hohlkörper mit im wesentlichen kreisförmigem Querschnitt besteht und zwei rohrförmige Stutzen als Lufteintritts- bzw. -austrittsöffnung aufweist.

Bei derartigen bekannten Pumpelementen (US-A-3 046 978) ist der Hohlkörper ein- oder mehrwandig ausgebildet und im oder am als Lufteintrittsöffnung dienenden Stutzen ein als Saugventil wirkendes Ventilelement angeordnet. An den anderen Stutzen, die Luftaustrittsöffnung, ist eine zur zu beatmenden Person, dem Patienten, führende Leitung angeschlossen, in die ein Dreiwegeventil, das Patientenventil, eingefügt ist. Bei künstlicher Beatmung wird durch manuelles Zusammendrücken des Hohlkörpers Luft oder Gas aus demselben über die Luftaustrittsöffnung und die Leitung mit dem Patientenventil, in die Lunge des Patienten gepreßt. Dabei ist das Saugventil in der Lufteintrittsöffnung des Hohlkörpers geschlossen. Wird der zusammengedrückte Hohlkörper entlastet, nimmt dieser aufgrund seiner selbstexpandierenden Eigenschaften seine ursprüngliche Gestalt wieder an und füllt sich über das Saugventil mit Luft aus der Umgebung oder einem anderen Gas, das aus einem Speicher der Lufteintrittsöffnung zugeführt wird. Dabei verhindert das Patientenventil, daß Gas oder Luft aus der Lunge des Patienten zurück in den Hohlkörper strömt, und schafft zugleich eine Verbindung mit der Atmosphäre, in die die ausgeatmete Luft abgeleitet wird.

Der Einsatz solcher Pumpelemente ist mit der Gefahr verbunden, daß dem Patienten mehr Luft zugeführt wird, als dessen Lunge während eines Atemzuges aufnehmen kann, wodurch dieser Person schwere Schäden durch eine druckmäßige Überlastung der Lunge zugefügt werden. Diese Gefahr ist besonders groß, wenn mit dem gleichen Pumpelement Patienten mit sehr verschiedenen Lungenvolumen wie Kinder und Erwachsene künstlich beatmet werden. Dieser Gefahr wird dadurch begegnet, daß die Pumpelemente mit Druckbegrenzern in Form von Überdruckventilen ausgerüstet sind. Überdruckventile sind jedoch sehr unzuverlässig und blockieren leicht, wodurch der druckbegrenzende Effekt entfällt. Ein anderer Nachteil dieser Druckbegrenzer besteht darin, daß der Öffnungsdruck zu niedrig eingestellt ist. Dies kann dazu führen, daß der Widerstand in den Luftwegen des Patienten nicht überwunden und somit keine Beatmung erhalten wird.

Eine andere Möglichkeit, Patienten während der künstlichen Beatmung vor Schäden zu bewahren, die von der Bedienung des Pumpelementes herrühren, ist die Begrenzung der während des Zusammendrückens des Pumpelementes abgegebenen Luftmenge. Bei dem in der US-PS-3 046 978 beschriebenen Pumpelement geschieht dies dadurch, daß in den Hohlkörper an bestimmten Stellen zwei elastische Kugeln verschiedener Größe angeordnet sind, die das Zusammendrücken des Hohlkörpers begrenzen. Der Hohlkörper trägt auf seiner Außenseite der Lage der Kugeln entsprechende Markierungen, die Griffzonen begrenzen. Jeder Griffzone ist somit ein bestimmtes Luftvolumen zugeordnet, das in etwa der Lungenkapazität von Kindern ; Jugendlichen und Erwachsenen entspricht. Diese Griffzonen sind nur optisch wahrzunehmen, was insbesondere bei der Beatmung von Personen an schlecht beleuchteten Unfallstellen zu fehlerhafter Handhabung des Pumpelementes und damit zur Schädigung des Patienten führen kann. Dieser Nachteil wird noch dadurch vergrößert, daß das Pumpelement doppelwandig ist und vor Gebrauch in den Raum zwischen seinen Wandungen Luft eingeblasen werden soll. Dies setzt voraus, daß der Innenbeutel des Pumpelementes vom Luftdruck im Zwischenraum nicht zusammengepreßt wird, was nur mit verhältnismäßig großen Selbstexpansionskräften in dessen Wandung zu erreichen ist. Diese Kräfte bewirken aber, daß sich das Pumpelement relativ schwer betätigen läßt, so daß dessen Benutzer den erzeugten Beatmungsdruck nicht fühlt, was zur Verhinderung einer sachgemäßen Beatmung des Patienten beiträgt.

In der FR-A-1 161 194 wird noch eine Vorrichtung zur künstlichen Beatmung beschrieben, die als Pumpelement einen Balg aufweist, der mit einem Handgriff gegen die Kraft einer Feder zusammengedrückt werden kann. Über dem als perforiertes Rohr ausgebildeten Handgriff ist eine Blase gasdicht befestigt, die beim Zusammendrücken des Balges, indem mit einer Hand auf den Handgriff eingewirkt wird, um eine Person zu beatmen, ebenfalls zusammengedrückt wird. Hierdurch wird die Luft aus der Blase über den Handgriff und eine Leitung dem Beatmungsventil zugeführt, um dessen Ventilkörper zu verschieben und so den Durchgang für die Beatmungsluft aus dem Balg in die Lunge des Patienten freizugeben. Die Wandung der aus Gummi bestehenden Blase ist mit sechs gleichmäßig über deren Umfang angeordneten Verstärkungsrippen versehen, die ein schnelles Aufblähen der mit einer Hand zusammengedrückten Blase bewirken, nachdem der Handgriff und damit die Blase losgelassen wurde, um den Bal einen neuen Atemvorgang zu füllen. Diese Druckentlastung der Blase hat zur Folge, daß der Ventilkörper des Beatmungsventiles in seine Ausgangsstellung zurückkehrt, so daß der Patient über das Beatmungsventil ausatmen kann. Dieser Vorrichtung haften ebenfalls die vorstehend geschilderten Nachteile an, da die dem Patienten aus dem Balg zugeführte Luft mengenmäßig nicht gesteuert werden kann.

Aufgabe der Erfindung ist es nun, ein handbetätigtes Pumpelement einer Vorrichtung zur künstlichen Beatmung von Menschen so auszubilden, daß es sowohl bei der Behandlung von Kindern als auch Erwachsenen ohne das Risiko einer druckmäßigen Überlastung von deren Lungen eingesetzt werden kann.

Diese Aufgabe wird erfindungsgemäß ausgehend von einem Pumpelement der eingangs beschriebenen Gattung dadurch gelöst, daß in die Wandung des Hohlkörpers zwei sich in axialer Richtung erstreckende Einbuchtungen eingearbeitet sind, die auf den Querschnitt des Hohlkörpers bezogen etwa 100 bis 140 Winkelgrad vorzugsweise etwa 120 Winkelgrad voneinander entfernt sind und zwei Griffzonen unmittelbar festlegen. Jede dieser Einbuchtungen ist zweckmäßigerweise so groß, daß der Daumen oder sämtliche Spitzen der anderen Finger einer Hand in sie eingreifen können.

Die Handhabung des Pumpelementes ist denkbar einfach ; denn der Hohlkörper muß nur so ergriffen werden, daß der Daumen und die Spitzen der anderen Finger einer Hand in je einer Einbuchtung liegen. Durch Zusammenballen oder Öffnen der Hand wird der Hohlkörper, um den Patienten mit Luft zu versorgen, zusammengedrückt und entleert bzw. aufgrund seiner selbstexpandierenden Eigenschaften aufgebläht und wieder mit unverbrauchter Luft gefüllt. Dabei kann der Hohlkörper so ergriffen werden, daß Handfläche und Finger entweder etwa 120 Winkelgrad oder 240 Winkelgrad des Mantelumfanges des Hohlkörpers überspannen, wodurch beim Zusammendrücken des Hohlkörpers dieser zu etwa einem Drittel oder zwei Dritteln entleert wird. Das Volumen des Hohlkörpers ist dabei so bemessen, daß die kleinere abgegebene Luftmenge an die Atemkapazität eines Kindes und die größere Luftmenge an das Lungenvolumen einer erwachsenen Person angepaßt ist. Da die zwei Möglichkeiten zum Erfassen des Hohlkörpers sich auffällig unterscheiden und die Finger der pumpenden Hand in den Einbuchtungen des Hohlkörpers gut geführt sind, wird ohne großen technischen Aufwand auf einfachste Weise sichergestellt, daß die Lunge eines Patienten während der künstlichen Beatmung nicht geschädigt wird.

Zur Vermeidung von möglicherweise kritischen Störungen während der künstlichen Beatmung sollen die Selbstexpansionskräfte des Hohlkörpers so groß sein, daß dieser sich nach dem Zusammendrücken mit einer mindestens der Atemfrequenz entsprechenden Geschwindigkeit auch dann entfaltet, wenn ein Bedienungsfehler das Entfalten verzögert. Andererseits ist es erwünscht, die Wandung des Hohlkörpers so dünn wie möglich zu bemessen, um insbesondere geübten Personen, den von ihnen beim Zusammendrücken des Hohlkörpers erzeugten Beatmungs- oder Ventilationsdruck fühlen zu lassen. Um dies zu erreichen, hat es sich bewährt, in die Wandung des Hohlkörpers sich zu den Einbuchtungen parallel erstreckende Vertiefungen und/oder Wülste einzuarbeiten.

Ausführungsbeispiele der Erfindung werden noch an Hand der Zeichnungen beschrieben. Es stellen dar :

Figur 1 eine schematische Ansicht einer Vorrichtung zur künstlichen Beatmung von Menschen mit einem handbetätigten Pumpelement nach der Erfindung,

Figur 2 eine schematische Schnittansicht längs der Linie I-I durch das Pumpelement nach Fig. 1,

Figur 3 eine Ansicht gemäß Fig. 2 durch ein anderes Pumpelement,

Figur 4 eine Ansicht gemäß Fig. 2 durch ein weiteres Pumpelement.

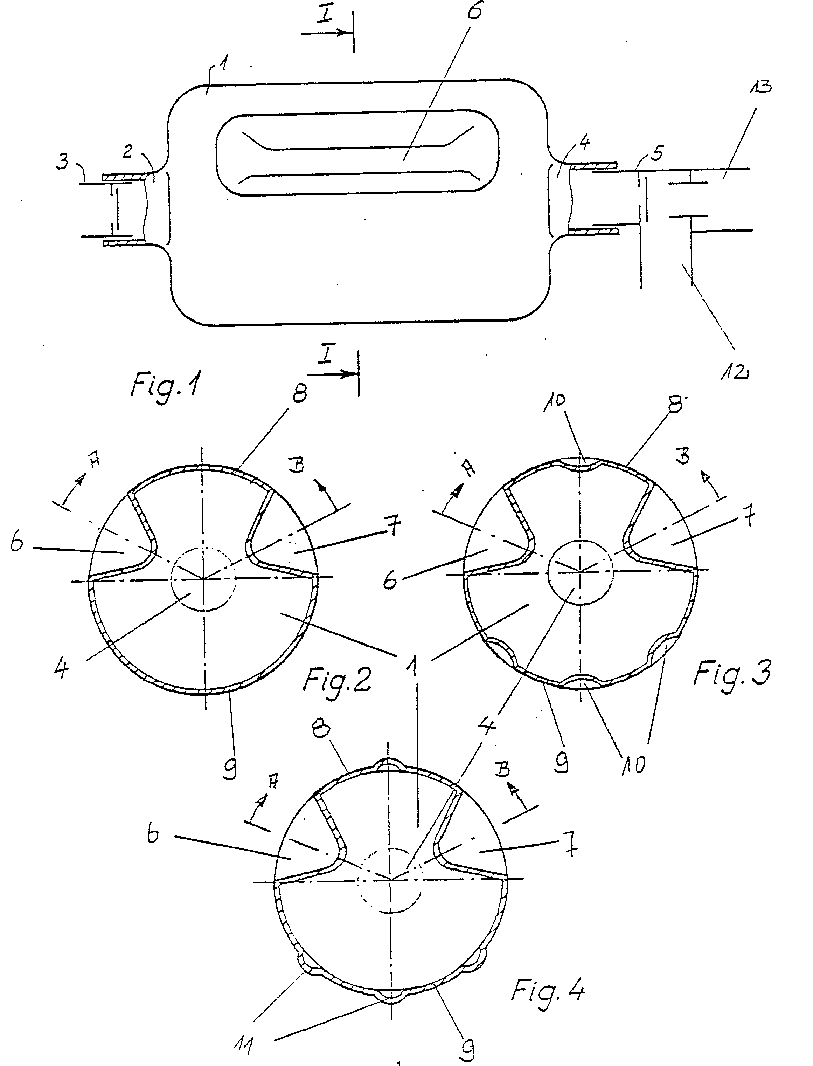

Die in Fig. 1 gezeigte Vorrichtung zur künstlichen Beatmung von Menschen weist als Pumpelement einen im wesentlichen zylindrischen Hohlkörper 1 auf, der aus einem gasdichten, elastischen Werkstoff mit selbstexpandierenden Eigenschaften gefertigt ist. An den Hohlkörper 1 sind zwei Stutzen 2 bzw. 4 als Lufteintritts- bzw. Luftaustrittsöffnungen angearbeitet. An den Stutzen 2 ist ein Saugventil 3 angeschlossen, während der Stutzen 4 mit einem Patientenventil 5 in Verbindung steht. In die Wandung des Hohlkörpers 1 sind zwei sich axial erstreckende Einbuchtungen 6 und 7 eingearbeitet, die bezogen auf den Querschnitt des Hohlkörpers etwa 120 Winkelgrad voneinander entfernt sind (Winkel AB, Fig. 2, 3 und 4).

Zur Verbesserung der selbstexpandierenden Eigenschaften des Hohlkörpers 1 kann dessen Wandung sich parallel zu den Einbuchtungen 6 und 7 erstreckende Vertiefungen 10 (Fig. 3) und/ oder Wülste 11 (Fig. 4) aufweisen.

Im folgenden wird noch kurz die Handhabung und Funktion des dargestellten Pumpelementes erläutert :

Wird der Hohlkörper 1 mit einer Hand so erfaßt, daß die Handfläche sich über den Abschnitt 8 des Hohlkörpers 1 erstreckt und der Daumen sowie die Spitzen der anderen Finger in die Einbuchtungen 6 bzw. 7 greifen, kann beim Zusammenballen der Hand zu einem Drittel der im Hohlkörper 1 vorhandenen Luft aus diesem heraus- und über den Stutzen 4 und den Ausgang 12 des Patientenventils 5 in die Lunge des Patienten gepreßt werden. Dabei ist das Saugventil 3 geschlossen und beträgt die vom Hohlkörper 1 abgegebene Luftmenge etwa 200 bis 400 cm³. Diese Luftmenge ist so gering, daß während der Beatmung eine Schädigung der Lunge eines Kindes nicht zu befürchten ist. Beim Öffnen der Hand entfaltet sich der Hohlkörper 1 und füllt sich über das Saugventil 3 wieder mit Luft. Während dieser Zeit atmet der Patient über den Ausgang 12 des Patientenventils 5 aus, das ein Rückströmen der verbrauchten Luft in den Hohlkörper 1 verhindert und diese über den Anschluß 13 an die Umgebung ableitet.

Erstreckt sich die Fläche der den Hohlkörper 1 ergreifenden Hand über dessen Abschnitt 9, wird beim Zusammenballen der Hand eine Luftmenge von 800 bis 1 000 cm³ dem Patienten über das Patientenventil 5 zugeführt. Dies ist die Luftmenge, die eine erwachsene Person während jedes Atemzuges bei künstlicher Beatmung ohne Gefahr für die Lunge aufnehmen kann.

Der das Pumpelement bildende Hohlkörper 1 kann erfingungsgemäß nicht nur die in den Figuren beschriebene Gestalt haben, sondern z. B. auch als Kugel oder Ellipsoid ausgebildet sein, sofern die Einbuchtungen 6 und 7 so angeordnet sind, daß bei der Handhabung des Hohlkörpers 1 in der beschriebenen Weise dieser entweder etwa zu einem Drittel oder zwei Dritteln entleert wird.

### Patentansprüche

1. Handbetätigtes Pumpelement einer Vorrichtung zur künstlichen Beatmung von Menschen, das aus einem gasdichten, elastischen, selbstexpandierenden und vorzugsweise langgestreckten Hohlkörper (1) mit im wesentlichen kreisförmigem Querschnitt besteht und zwei rohrförmige Stutzen (2, 4) als Lufteintritts- bzw. -austrittsöffnung aufweist, dadurch gekennzeichnet, daß in die Wandung des Hohlkörpers (1) zwei sich in axialer Richtung erstreckende Einbuchtungen (6, 7) eingearbeitet sind, die auf den Querschnitt des Hohlkörpers (1) bezogen etwa 100 bis 140 Winkelgrad, vorzugsweise etwa 120 Winkelgrad, voneinander entfernt sind.

2. Pumpelement nach Anspruch 1, dadurch gekennzeichnet, daß jede der Einbuchtungen (6, 7) so groß ist, daß der Daumen oder sämtliche Spitzen der anderen Finger einer Hand in dieselben (6, 7) eingreifen können.

3. Pumpelement nach Anspruch 1 oder 2, dadurch gekennzeichnet, daß in die Wandung des Hohlkörpers (1) sich zu den Einbuchtungen (6, 7) parallel erstreckende Vertiefungen (10) und/oder Wülste (11) eingearbeitet sind.

### Claims

1. A manually operated pump element of an artificial breathing device for human beings, which consists of a gas-tight, resilient, self-expanding and preferably elongated hollow body (1) with a substantially circular cross-section and comprises two tubular sockets (2, 4) as an air inlet and air outlet respectively, characterised in that formed in the wall of the hollow body (1) are two depressions (6, 7) which extend in the axial direction and which are at a distance apart of about 100 to 140 angular degrees, preferably about 120 angular degrees, in relation to the cross-section of the hollow body (1).

2. A pump element according to claim 1, characterised in that each of the depressions (6, 7) is so large that the thumb or all the tips of the other fingers of one hand can engage therein (6, 7).

3. A pump element according to claim 1 or 2, characterised in that recesses (10) and/or beads (11) extending parallel to the depressions (6, 7) are formed in the wall of the hollow body (1).

### Revendications

1. Elément de pompe actionné à la main d'un dispositif pour la respiration artificielle humaine, constitué par un corps creux (1) étanche aux gaz, élastique, à expansion spontanée et de préférence de forme allongée, et comporte deux ajutages tubulaires (2, 4) formant respectivement ouverture d'entrée et ouverture de sortie d'air, caractérisé en ce que deux renforcements (6, 7) s'étendant axialement, sont ménagés dans la paroi du corps creux (1) et vus en section transversale du corps creux (1), sont distants l'un de l'autre d'environ 100 à 140 degrés, de préférence d'environ 120 degrés.

2. Elément de pompe selon la revendication 1, caractérisé en ce que chacun des renforcements (6, 7) est de dimension suffisante pour que le pouce ou les extrémités de tous les autres doigts d'une main puissent s'y engager.

3. Elément de pompe selon la revendication 1 ou 2, caractérisé en ce que la paroi du corps creux (1) comporte des enfoncements (10) et/ou des bourrelets (11) s'étendant parallèlement aux renforcements (6, 7).

Fig. 1

Fig. 2

Fig. 3

Fig. 4